(19) Europäisches Patentamt / European Patent Office / Office européen des brevets

(11) EP 2 739 146 B1

(12) **EUROPÄISCHE PATENTSCHRIFT**

(45) Veröffentlichungstag und Bekanntmachung des Hinweises auf die Patenterteilung:
**25.11.2015 Patentblatt 2015/48**

(21) Anmeldenummer: **12741261.7**

(22) Anmeldetag: **31.07.2012**

(51) Int Cl.:
***A01N 43/90*** (2006.01) ***A01P 1/00*** (2006.01)
***A61K 31/34*** (2006.01) ***A61K 8/49*** (2006.01)
***A61Q 17/00*** (2006.01) ***A61Q 19/00*** (2006.01)
***A61K 45/06*** (2006.01) ***A61K 31/047*** (2006.01)
***A61K 31/341*** (2006.01)

(86) Internationale Anmeldenummer:
**PCT/EP2012/003246**

(87) Internationale Veröffentlichungsnummer:
**WO 2013/017257 (07.02.2013 Gazette 2013/06)**

(54) **VERWENDUNG VON ISOSORBIDMONOESTERN ALS FUNGIZIDE WIRKSTOFFE**

USE OF ISOSORBIDE MONOESTERS AS FUNGICIDALLY ACTIVE SUBSTANCES

UTILISATION DE MONOESTERS D'ISOSORBIDE EN TANT QUE PRINCIPES ACTIFS FONGICIDS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2011 DE 102011109435**

(43) Veröffentlichungstag der Anmeldung:
**11.06.2014 Patentblatt 2014/24**

(73) Patentinhaber: **CLARIANT INTERNATIONAL LTD
4132 Muttenz (CH)**

(72) Erfinder:
- **PILZ, Maurice, Frederic
60329 Frankfurt am Main (DE)**
- **KLUG, Peter
63762 Großostheim (DE)**
- **SCHERL, Franz-Xaver
84508 Burgkirchen (DE)**
- **GROHMANN, Joerg
65527 Niedernhausen (DE)**

(74) Vertreter: **Holmes, Rosalind et al
Clariant Produkte (Deutschland) GmbH
Patent & License Management Chemicals
Industriepark Höchst, G 860
65926 Frankfurt am Main (DE)**

(56) Entgegenhaltungen:
**WO-A2-2010/108738 WO-A2-2010/136121
JP-A- 8 187 070**

- **PETER STOSS ET AL: "Regioselektive Acylierung von 1,4:3,6-Dianhydro-D-glucit", SYNTHESIS, Bd. 1987, Nr. 02, 1. Januar 1987 (1987-01-01), Seiten 174-176, XP55039551, ISSN: 0039-7881, DOI: 10.1055/s-1987-27878**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 739 146 B1

## Beschreibung

Die vorliegende Erfindung betrifft die Verwendung von Isosorbidmonoestern als Fungizid.

In der Industrie werden Konservierungsmittel oder Biozide verwendet, um Produkte wie beispielsweise kosmetische, dermatologische oder pharmazeutische Zusammensetzungen, Pflanzenschutzformulierungen, Wasch- oder Reinigungsmittel oder Farb- oder Anstrichmittel gegen mikrobiellen Befall zu schützen. Es sind zahlreiche Konservierungsmittel oder Biozide für diesen Zweck bekannt. Beispielsweise können Konservierungsmittel aus Annex V der EU-Kosmetik-Richtlinie oder Biozide der EU-Biozid-Richtlinie hierfür verwendet werden.

Nachteilig an der Verwendung vieler Konservierungsmittel ist jedoch, dass ihre Herstellung oftmals aufwändig ist und auf synthetischen Rohstoffen basiert. Zudem ist ihre konservierende Wirkung oftmals verbesserungsbedürftig, so dass hohe Einsatzkonzentrationen für eine ausreichende Konservierung notwendig sind.

Es bestand deshalb die Aufgabe, antimikrobielle Wirkstoffe zur Verfügung zu stellen, die eine vorteilhafte Konservierungsmittelleistung aufweisen und sich zudem durch den Vorteil auszeichnen, dass sie auf nachwachsenden Rohstoffen basieren.

Überraschend wurde nun gefunden, dass diese Aufgabe gelöst wird durch Verbindungen der Formel (I)

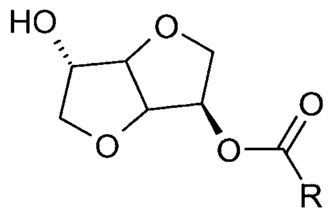

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist.

Gegenstand der Erfindung ist somit die Verwendung einer oder mehrerer Verbindungen der Formel (I)

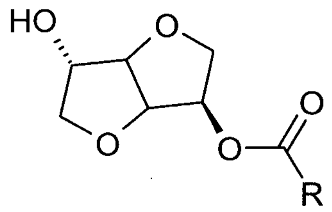

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, als Fungizid.

Die Verbindungen der Formel (I) weisen eine sehr gute Konservierungsmittelleistung auf und sind zur Verwendung als Fungizide geeignet. Sie basieren außerdem auf nachwachsenden Rohstoffen.

Im Vergleich zur Verwendung von organischen Säuren als Konservierungsmittel besitzen die Verbindungen der Formel (I) zudem den Vorteil einer Wirksamkeit über einen breiteren pH-Wert-Bereich. Während organische Säuren oftmals eine gute Wirkung nur im pH-Wert-Bereich von 3,5 bis 6 zeigen, können die Verbindungen der Formel (I) auch bei höheren pH-Werten vorteilhaft eingesetzt werden.

Zusammensetzungen, die zumindest zum Teil auf nachwachsenden Rohstoffen basieren und als Konservierungsmittel verwendet werden können, sind bereits bekannt.

DE 10 2009 022 444 (Clariant) beschreibt flüssige Zusammensetzungen enthaltend Sorbitanmonocaprylat und antimikrobielle Wirkstoffe wie z. B. spezielle organische Säuren und ihre Salze, spezielle Formaldehyd-Donoren, spezielle Isothiazolinone, spezielle Parabenester und ihre Salze und spezielle Pyridone und ihre Salze sowie ihre Verwendung zum Konservieren von kosmetischen, dermatologischen oder pharmazeutischen Produkten.

In der DE 10 2009 022 445 (Clariant) werden flüssige Zusammensetzungen enthaltend Sorbitanmonocaprylat und Alkohol und ihre Verwendung zum Konservieren von kosmetischen, dermatologischen oder pharmazeutischen Produkten offenbart.

In der JP 8187070 (A) (Lion) wird eine Mischung aus Fettsäuremonoestern von $C_8$-$C_{18}$ Fettsäuren und mindestens einem Polyol ausgewählt aus Sorbitol, 1,5-Sorbitan, 1,4-Sorbitan und Isosorbid und Fettsäurediestern dieser Fettsäuren und Polyole in einem Gewichtsverhältnis von Monoester: Diester von 33 : 7 bis 9 : 1 als antimikrobieller Wirkstoff gegen Bakterien für Nahrungsmittel oder Getränke offenbart.

Verbindungen der Formel (I) können z. B. nach dem Fachmann geläufigen Methoden hergestellt werden. Beispielsweise können die Verbindungen der Formel (I) durch Veresterung von Isosorbid nach üblichen und dem Fachmann bekannten Methoden hergestellt werden, wobei sowohl Isosorbid selbst als auch die zur Veresterung verwendeten Säurekomponenten wiederum käuflich erwerbbar sind.

Die eine oder die mehreren Verbindungen der Formel (I) werden als Fungizid verwendet. Dies bedeutet im Rahmen der vorliegenden Anmeldung, dass die eine oder die mehreren Verbindungen der Formel (I) vorzugsweise als antimikrobieller Wirkstoff gegenüber Hefen und Pilzen verwendet werden können. Besonders bevorzugt wird die eine oder werden die mehreren Verbindungen der Formel (I) als antimikrobieller Wirkstoff gegenüber Pilzen verwendet.

Vorzugsweise ist der Rest R in der einen oder den mehreren Verbindungen der Formel (I) ein linearer gesättigter Alkylrest mit 7 bis 9 Kohlenstoffatomen. Besonders bevorzugt ist der Rest R in der einen oder den mehreren Verbindungen der Formel (I) ein linearer gesättigter Alkylrest mit 7 Kohlenstoffatomen.

Die eine oder die mehreren Verbindungen der Formel (I) können alleine oder in Zusammensetzungen enthaltend eine oder mehrere andere Substanzen erfindungsgemäß als Fungizid verwendet werden. Diese Zusammensetzungen werden im Folgenden als "Zusammensetzungen A" bezeichnet.

In einer bevorzugten Ausführungsform der Erfindung enthalten die Zusammensetzungen A eine oder mehrere Verbindungen der Formel (I) und zusätzlich eine oder mehrere andere Substanzen ausgewählt aus der Gruppe bestehend aus Sorbitol, Sorbitolestern (bei Sorbitolestern kann es sich um Mono-, Di-, Tri-, Tetra-, Penta- und/oder Hexaester handeln), Sorbitan, Sorbitanestern (bei Sorbitanestern kann es sich um Mono-, Di-, Tri- und/oder Tetraester handeln), Isosorbid, Isosorbiddiestern und Carbonsäuren. Bei "Sorbitan" kann es sich beispielsweise um 1,4- oder 1,5-Sorbitan handeln. Sowohl die Carbonsäuren selbst als auch die den Säurekomponenten der genannten Ester zu Grunde liegenden Carbonsäuren entsprechen der Formel RCOOH, worin R die bei Formel (I) angegebene Bedeutung besitzt und vorzugsweise ein linearer gesättigter Alkylrest mit 7 Kohlenstoffatomen ist, d.h. die Carbonsäure RCOOH vorzugsweise Caprylsäure ist.

In einer besonders bevorzugten Ausführungsform der Erfindung enthalten die Zusammensetzungen A eine oder mehrere Verbindungen der Formel (I) und zusätzlich

I) Isosorbid und
II) einen oder mehrere Isosorbiddiester der Formel (II)

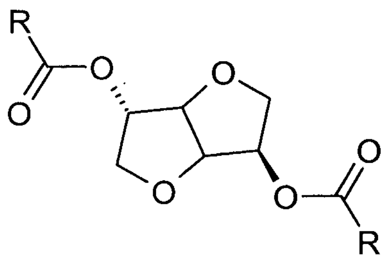

(II)

worin R die oben bei Formel (I) angegebene Bedeutung besitzt, und wobei der Isosorbiddiester vorzugsweise Isosorbiddicaprylat ist.

Hierunter wiederum bevorzugt enthalten die soeben genannten Zusammensetzungen A eine oder mehrere Verbindungen der Formel (I) und zusätzlich

I) 0,05 bis 0,7, vorzugsweise 0,1 bis 0,7 und besonders bevorzugt 0,2 bis 0,5 Gewichtsteile Isosorbid und
II) 0,1 bis 1,0, vorzugsweise 0,2 bis 1,0 und besonders bevorzugt 0,4 bis 0,8 Gewichtsteile an dem einen oder den mehreren Isosorbiddiestern der Formel (II), wobei der Isosorbiddiester vorzugsweise Isosorbiddicaprylat ist,

jeweils bezogen auf 1,0 Gewichtsteile an der einen oder den mehreren Verbindungen der Formel (I) und vorzugsweise bezogen auf 1,0 Gewichtsteile an Isosorbidmonocaprylat.

In einer hierunter wiederum bevorzugten Ausführungsform der Erfindung enthalten die soeben genannten Zusammensetzungen A entweder keine Carbonsäure RCOOH oder bis zu 0,1, vorzugsweise 0,001 bis 0,05 und besonders bevorzugt 0,002 bis 0,01 Gewichtsteile Carbonsäure RCOOH, wobei R die oben bei Formel (I) angegebene Bedeutung besitzt, und wobei die Carbonsäure vorzugsweise Caprylsäure ist, bezogen auf 1,0 Gewichtsteile an der einen oder den mehreren Verbindungen der Formel (I) und vorzugsweise bezogen auf 1,0 Gewichtsteile an Isosorbidmonocaprylat.

In einer weiteren besonders bevorzugten Ausführungsform der Erfindung enthalten die Zusammensetzungen A eine oder mehrere Verbindungen der Formel (I) und einen oder mehrere Sorbitanester aus Sorbitan und Carbonsäuren $R^aCOOH$, vorzugsweise ausgewählt aus Sorbitanestern aus 1,4- und/oder 1,5-Sorbitan und Carbonsäuren $R^aCOOH$, wobei $R^a$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, und wobei das Gewichtsverhältnis der einen oder der mehreren Verbindungen der Formel (I) zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist. Das angegebene Gewichtsverhältnis von "100 : 0" bedeutet, dass die soeben genannten Zusammensetzungen A in dieser besonders bevorzugten Ausführungsform der Erfindung keinen Sorbitanester enthalten müssen.

Unter den soeben genannten Zusammensetzungen A sind solche bevorzugt, worin der eine oder die mehreren Sorbitanester aus Sorbitan und Carbonsäuren $R^aCOOH$ ausgewählt sind aus Sorbitanestern aus Sorbitan und Caprylsäure und vorzugsweise ausgewählt sind aus Sorbitanestern aus 1,4- und/oder 1,5-Sorbitan und Caprylsäure und der Sorbitanester besonders bevorzugt Sorbitanmonocaprylat ist.

In diesen Zusammensetzungen A ist die OH-Zahl der Mischung aus der einen oder den mehreren Verbindungen der Formel (I) und der einen oder den mehreren (gegebenenfalls enthaltenen) Sorbitanestern aus Sorbitan und Carbonsäuren $R^aCOOH$ vorzugsweise kleiner oder gleich 320, besonders bevorzugt kleiner oder gleich 285, insbesondere bevorzugt kleiner oder gleich 245 und außerordentlich bevorzugt kleiner oder gleich 225.

In einer weiteren besonders bevorzugten Ausführungsform der Erfindung ist die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren in den Zusammensetzungen A kleiner oder gleich 320, vorzugsweise kleiner oder gleich 285, besonders bevorzugt kleiner oder gleich 245 und insbesondere bevorzugt kleiner oder gleich 225.

In einer weiteren besonders bevorzugten Ausführungsform der Erfindung enthalten die Zusammensetzungen A keine Verbindungen ausgewählt aus Sorbitol und Sorbitolestern.

In einer weiteren besonders bevorzugten Ausführungsform der Erfindung enthalten die Zusammensetzungen A keine Verbindungen ausgewählt aus Sorbitan und Sorbitanestern.

Sofern die Zusammensetzungen A eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 5,0 Gew.-%, besonders bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder gleich 0,5 Gew.-% in den Zusammensetzungen A enthalten, wobei die Angaben in Gew.-% jeweils auf das Gesamtgewicht der fertigen Zusammensetzungen A bezogen sind.

Sofern die Zusammensetzungen A eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 20,0 Gew.-%, besonders bevorzugt in einer Menge kleiner oder gleich 10,0 Gew.-%, insbesondere bevorzugt in einer Menge kleiner oder gleich 5,0 Gew.-% und außerordentlich bevorzugt in einer Menge kleiner oder gleich 1,0 Gew.-% in den Zusammensetzungen A enthalten, wobei die Angaben in Gew.-% jeweils auf das Gesamtgewicht der fertigen Zusammensetzungen A bezogen sind.

In einer weiteren besonders bevorzugten Ausführungsform der Erfindung enthalten die Zusammensetzungen A die eine oder die mehreren Verbindungen der Formel (I) in Mengen von mindestens 30 Gew.-%, vorzugsweise in Mengen von mindestens 50 Gew.-% und besonders bevorzugt in Mengen von mindestens 60 Gew.-%, jeweils bezogen auf das Gesamtgewicht der fertigen Zusammensetzungen A.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt)

werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

| | |
|---|---|
| OHZ | = Hydroxylzahl in mg KOH/g Substanz |
| Va | = Verbrauch an Natronlauge in ml bei der Titration der Probe |
| Vb | = Verbrauch an Natronlauge in ml bei der Titration des Wirkwertes |
| c | = Stoffmengenkonzentration der Natronlauge in mol/l |
| t | = Titer der Natronlauge |
| M | = Molare Masse von KOH = 56,11 g/mol |
| E | = Probeneinwaage in g |

(Vb - Va) ist diejenige Menge der verwendeten Natronlauge in ml, die der bei der oben beschriebenen Acetylierung der zu messenden Probe gebundenen Menge an Essigsäure äquivalent ist.

Die soeben beschriebene Methode zur Bestimmung der OH-Zahl wird im Folgenden als "Methode OHZ-A" bezeichnet.

Vorzugsweise findet die erfindungsgemäße Verwendung in kosmetischen, dermatologischen oder pharmazeutischen Zusammensetzungen, in Pflanzenschutzformulierungen, in Wasch- oder Reinigungsmitteln oder in Farb- oder Anstrichmitteln statt. Die Pflanzenschutzmittel enthalten vorzugsweise ein oder mehrere Pestizide.

Die kosmetischen, dermatologischen oder pharmazeutischen Zusammensetzungen, die Pflanzenschutzformulierungen, die Wasch- oder Reinigungsmittel oder die Farb- oder Anstrichmittel enthalten die eine oder die mehreren Verbindungen der Formel (I) vorzugsweise in Mengen von 0,01 bis 10,0 Gew.-%, besonders bevorzugt in Mengen von 0,1 bis 5,0 Gew.-% und insbesondere bevorzugt in Mengen von 0,2 bis 3,0 Gew.-%, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

Die kosmetischen, dermatologischen oder pharmazeutischen Zusammensetzungen, die Pflanzenschutzformulierungen, die Wasch- oder Reinigungsmittel oder die Farb- oder Anstrichmittel haben Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz bei 20 Umdrehungen pro Minute).

Die kosmetischen, dermatologischen oder pharmazeutischen Zusammensetzungen liegen vorzugsweise in Form von Fluids, Gelen, Schäumen, Sprays, Lotions oder Cremes vor.

Bei den kosmetischen, dermatologischen oder pharmazeutischen Zusammensetzungen handelt es sich vorzugsweise um Mittel zur Behandlung von Fußpilz oder um Antischuppenmittel. Die erfindungsgemäße Verwendung findet in diesen Fällen als Fungizid gegen Fußpilz und gegen Schuppen statt.

Die kosmetischen, dermatologischen oder pharmazeutischen Zusammensetzungen, die Pflanzenschutzformulierungen, die Wasch- oder Reinigungsmittel oder die Farb- oder Anstrichmittel sind vorzugsweise auf wässriger oder wässrig-alkoholischer Basis aufgebaut oder liegen als Emulsionen oder Dispersionen vor. Besonders bevorzugt liegen sie als Emulsionen vor und insbesondere bevorzugt liegen sie als Öl-in-Wasser Emulsionen vor.

Die kosmetischen, dermatologischen oder pharmazeutischen Zusammensetzungen, die Pflanzenschutzformulierungen, die Wasch- oder Reinigungsmittel oder die Farb- oder Anstrichmittel können als weitere Hilfs- und Zusatzstoffe alle üblicherweise für die jeweilige Anwendung üblicherweise verwendeten Substanzen enthalten, beispielsweise Öle, Wachse, Emulgatoren, Co-Emulgatoren, Dispergatoren, Tenside, Entschäumer, Solubilisatoren, Elektrolyte, Hydroxysäuren, Stabilisatoren, Polymere, Filmbildner, Verdicker, Gelierungsmittel, Überfettungsmittel, Rückfetter, weitere antimikrobielle Wirkstoffe, biogene Wirkstoffe, Adstringentien, Aktivstoffe, deodorierende Stoffe, Sonnenschutzfilter, Antioxidantien, Oxidantien, Feuchthaltemittel, Lösungsmittel, Farbmittel, Pigmente, Perlglanzmittel, Duftstoffe, Trübungsmittel und/oder Silikone enthalten.

Die kosmetischen, dermatologischen oder pharmazeutischen Zusammensetzungen, die Pflanzenschutzformulierungen, die Wasch- oder Reinigungsmittel oder die Farb- oder Anstrichmittel besitzen pH-Werte von vorzugsweise 2 bis 11, besonders bevorzugt von 4,5 bis 8,5 und insbesondere bevorzugt von 5,5 bis 6,5.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

Versuchsbeispiele:

A) Herstellung von Isosorbidcaprylat

In einer Rührapparatur mit Destillationsaufsatz werden 190,0 g (1,3 mol) Isosorbid ("Sorbon" von Ecogreen Oleochemicals) und 187,5 g (1,3 mol) Octansäure (Caprylsäure) bei 80 °C zusammen mit 0,38 g Natronlauge (18 Gew.-%ig, wässrig) als Katalysator vorgelegt. Unter Rühren und Stickstoffüberleitung (10 - 12 Liter pro Stunde) wird das Reaktionsgemisch zunächst auf 180 °C aufgeheizt, wobei das Reaktionswasser abzudestillieren beginnt. Der Ansatz wird dann in 1 Stunde auf 190 °C und in weiteren 2 Stunden auf 210 °C aufgeheizt. Nach Erreichen von 210 °C wird solange verestert bis eine Säurezahl von < 1 mg KOH/g erreicht ist. Man erhält 345,7 g bernsteinfarbenes Isosorbidcaprylat (97 % der Theorie). Der pH-Wert (5 Gew.-% in Ethanol/Wasser 1:1) beträgt 5,9. Der pH-Wert wurde gemessen gemäß DIN EN 1262.

Weitere analytische Kenndaten des Isosorbidcaprylats:

| | |
|---|---|
| Säurezahl: | 0,9 mg KOH/g, gemessen gemäß DIN EN ISO 2114 |
| Hydroxyl-Zahl: | 206 mg KOH/g, gemessen in Anlehnung an DIN 53240-2 nach Methode OHZ-A |
| Verseifungszahl: | 204 mg KOH/g, gemessen gemäß DIN EN ISO 3681 |

Das Isosorbidcaprylat besitzt folgende Zusammensetzung:

| Substanz | Gew.-% |
|---|---|
| Caprylsäure | 0,4 |
| Isosorbid | 18,1 |
| Isosorbidmonocaprylat | 50,9 |
| Isosorbiddicaprylat | 30,6 |

Diese Zusammensetzung wird im Folgenden als "Isosorbidcaprylat 1" bezeichnet.

B) Bestimmung der antimikrobiellen Wirksamkeit von Isosorbidcaprylat 1

Im Folgenden wird die antimikrobielle Wirksamkeit von Isosorbidcaprylat 1 in Butylpolyglykol gegen Bakterien, Pilze und Hefen untersucht. Für die Ausprüfung mit Bakterien wurde Isosorbidcaprylat 1 mit Butylpolyglykol verdünnt und anschließend zu flüssigem, auf pH 7 (+/- 0,2) gepufferten Caso-Agar (Casein-Pepton-Agar) bei 50 °C in unterschiedlichen Konzentrationen gegeben (im Folgenden Zusammensetzungen B1, B2, etc. genannt). Für die Ausprüfung mit Pilzen und mit Hefen wurde Isosorbidcaprylat 1 mit Butylpolyglykol verdünnt und anschließend zu flüssigem, auf pH 5,6 (+/- 0,2) gepufferten Sabouraud-4 %-Dextrose Agar in unterschiedlichen Konzentrationen gegeben (im Folgenden Zusammensetzungen PH1, PH2, etc. genannt). Jede der Zusammensetzungen B1, B2, etc. bzw. PH1, PH2 etc. wurde in Petrischalen ausgegossen und jeweils mit der gleichen Menge an Bakterien, Pilzen und Hefen beimpft. Die minimale Hemmkonzentration (MHK) ist die Konzentration, bei der eine Hemmung des Wachstums der Bakterien, Pilze und Hefen in den Zusammensetzungen B1, B2, etc. bzw. PH1, PH2, etc. auftritt.

Die ermittelten und im Folgenden in Tabelle 1 angegebenen Werte für die minimalen Hemmkonzentrationen von Isosorbidcaprylat 1 sind bereits um den Verdünnungseffekt des Butylpolyglykols bereinigt.

Tabelle 1 Minimale Hemmkonzentrationen (MHK) von Isosorbidcaprylat 1

| Untersuchte Bakterien (B), Pilze (P) oder Hefen (H) | MHK von Isosorbidcaprylat 1 [ppm] |
|---|---|
| *Staphylococcus aureus (B)* | 2500 |
| *Pseudomonas aeruginosa (B)* | 10000 |
| *Escherichia coli (B)* | 7500 |
| *Enterobacter aerogenes (B)* | 10000 |
| *Klebsiella pneumoniae (B)* | 10000 |
| *Proteus vulgaris (B)* | 5000 |
| *Pseudomonas oleovorans (B)* | 10000 |
| *Citrobacter freundii (B)* | 10000 |
| *Candida albicans (H)* | 600 |
| *Aspergillus brasiliensis (P)* | 800 |
| *Penicillium minioluteum (P)* | 600 |
| *Aspergillus terreus (P)* | 600 |
| *Fusarium solani (P)* | 600 |
| *Penicillium funicolosium (P)* | 400 |

An den in Tabelle 1 aufgeführten Ergebnissen erkennt man, dass Isosorbidcaprylat 1 eine antimikrobielle Wirksamkeit besitzt, insbesondere gegen die Hefe *Candida albicans* und die getesteten Pilze.

C) Antimikrobielle Wirksamkeit der Bestandteile von Isosorbidcaprylat 1

Caprylsäure besitzt eine antimikrobielle Wirksamkeit. Da Caprylsäure in der Zusammensetzung "Isosorbidcaprylat 1" aber nur zu 0,4 Gew.-% vorliegt, ist ihre antimikrobielle Wirksamkeit in dieser Zusammensetzung vernachlässigbar klein. Caprylsäure hat zudem bei pH-Werten von 6 oder größer keine antimikrobielle Wirksamkeit.

Analog zur Bestimmung der antimikrobiellen Wirksamkeit von Isosorbidcaprylat 1 wurde in weiteren Testreihen die antimikrobielle Wirksamkeit einer Mischung enthaltend 89,6 Gew.-% Isosorbiddicaprylat und 9,4 Gew.-% Isosorbidmonocaprylat (Rest: 1 Gew.-%) (im Folgenden "Isosorbiddicaprylat" genannt) einerseits und reinem Isosorbid andererseits bestimmt. Die Ergebnisse sind in Tabelle 2 dargestellt.

Tabelle 2 Minimale Hemmkonzentrationen (MHK) von Isosorbiddicaprylat und Isosorbid

| Untersuchte Bakterien (B), Pilze (P) oder Hefen (H) | MHK von Isosorbiddicaprylat [ppm] | MHK von Isosorbid [ppm] |
|---|---|---|
| *Staphylococcus aureus (B)* | 10000 | 10000 |
| *Pseudomonas aeruginosa (B)* | 10000 | 10000 |
| *Escherichia coli* (B) | 10000 | 10000 |
| *Enterobacter aerogenes (B)* | 10000 | 10000 |
| *Klebsiella pneumoniae (B)* | 10000 | 10000 |
| *Proteus vulgaris (B)* | 10000 | 10000 |
| *Pseudomonas oleovorans (B)* | 10000 | 10000 |
| *Citrobacter freundii (B)* | 10000 | 10000 |
| *Candida albicans (H)* | 10000 | 10000 |
| *Aspergillus brasiliensis (P)* | 10000 | 10000 |
| *Penicillium minioluteum (P)* | 10000 | 10000 |
| *Aspergillus terreus (P)* | 10000 | 10000 |

(fortgesetzt)

| Untersuchte Bakterien (B), Pilze (P) oder Hefen (H) | MHK von Isosorbiddicaprylat [ppm] | MHK von Isosorbid [ppm] |
|---|---|---|
| *Fusarium solani (P)* | 5000 | 10000 |
| *Penicillium funicolosium (P)* | 5000 | 10000 |

Wie aus den Ergebnissen der Tabelle 2 hervorgeht, besitzen weder Isosorbid noch Isosorbiddicaprylat eine antimikrobielle Wirksamkeit.

Auf Grund der fehlenden antimikrobiellen Wirksamkeit der in der Zusammensetzung Isosorbidcaprylat 1 enthaltenen Verbindungen Caprylsäure, Isosorbid und Isosorbiddicaprylat einerseits und der aus den Ergebnissen der Tabelle 1 ersichtlichen antimikrobiellen Wirksamkeit der Zusammensetzung "Isosorbidcaprylat 1" andererseits wird gefolgert, dass die ebenfalls in der Zusammensetzung Isosorbidcaprylat 1 enthaltene Verbindung Isosorbidmonocaprylat eine signifikante antimikrobielle Wirksamkeit, insbesondere als Fungizid gegenüber Hefen und Pilzen, besitzt.

Aus diesem Grund wird auch davon ausgegangen, dass die geringfügige Aktivität von der Zusammensetzung Isosorbiddicaprylat gegenüber den Pilzen *Fusarium solani* und *Penicillium funicolosium* auf die darin enthaltene Verbindung Isosorbidmonocaprylat zurückzuführen ist.

D) Anwendungsbeispiele

Die erfindungsgemäße Verwendung kann beispielsweise in folgenden Formulierungen stattfinden.

Formulierungsbeispiel 1: Revitalisierende Feuchtigkeitscreme

| Phase | Inhaltsstoff | Gew.-% |
|---|---|---|
| A | Hostacerin® SFO<br>Sunflower Seed Oil Sorbitol Esters<br>Velsan® CCT<br>Caprylic/Capric Triglyceride<br>Cetiol® OE<br>Dicaprylyl Ether<br>Lanette® 22<br>Benehyl Alcohol<br>Lanette® 18<br>Stearyl Alcohol<br>Fucogel® 1000<br>Biosacchride Gum-1 | 2,0<br><br>4,5<br><br>4,5<br><br>4,0<br><br>4,0<br><br>1,0 |
| B | Coenzyme® Q 10<br>Ubiquinone | 0,1 |
| C | Wasser<br>Glycerin<br>Hostaphat® CK 100<br>Potassium Cetyl Phosphate | ad 100<br>10,0<br>0,6 |
| D | Phenoxyethanol<br>Isosorbidcaprylat 1 | 1,0<br>1,0 |
| E | NaOH (10 Gew.-%ig in Wasser) | q.s. |

Herstellung:

I Mische die Komponenten von A und erwärme auf 80 °C
II Mische die Komponenten von C und erwärme auf 80 °C
III Gebe B zu I.
IV Gebe II zu III und rühre bis die Mischung auf Raumtemperatur abgekühlt ist
V Gebe D zu IV
VI Stelle den pH-Wert mit E auf pH 5,5 ein

Formulierungsbeispiel 2:

| Phase | Inhaltsstoff | Gew.-% |
|---|---|---|
| A | Hostacerin® EWO Polyglyceryl-2-Sesquiisostearate (and) Cera Alba (and) Carnauba Wax (and) Ethylhexylstearate (and) Magnesium Stearate (and) Aluminum Stearate | 16,0 |
| | Isopropyl Palmitate | 10,0 |
| | Avocado Oil | 2,0 |
| | Velsan® CCT | 2,5 |
| | Caprylic/Capric Tryglyceride | |
| B | Octopirox® | 0,05 |
| | Piroctone Olamine | |
| | Propylene Glycol | 1,0 |
| C | Wasser | ad 100 |
| | Glycerin | 4,0 |
| | Magnesium Sulfate * 7 $H_2O$ | 0,7 |
| | Allantoin | 0,5 |
| D | Tocopheryl Acetate | 0,5 |
| | Rosmarinus Officinalis (Rosemary) Leaf Oil | 0,1 |
| | Urea | 10,0 |
| | Isosorbidcaprylat 1 | 1,0 |
| | Phenoxyethanol | 0,8 |

Herstellung:

I Mische die Komponenten von A und erwärme auf 80 °C
II Mische die Komponenten von B bis alle Stoffe gelöst sind (eventuell unter leichtem Erwärmen)
III Gebe II zu I
IV Mische die Komponenten von C und erwärme auf 50 °C
V Rühre IV in I mit hoher Geschwindigkeit bis zur Abkühlung auf 35 °C
VI Gebe D bei 35 °C zu V

Formulierungsbeispiele 3 und 4: Pflanzenschutzformulierungen

| Formulierung Nr. | 3 | 4 |
|---|---|---|
| Inhaltsstoff | Menge des jeweiligen Inhaltsstoffes [Gew.-%] | |
| Atrazin | 43,6 | 43,6 |
| Dispersogen® PSL 100 | - | 1,7 |
| Genapol® LSS | - | 1,6 |

(fortgesetzt)

| Formulierung Nr. | 3 | 4 |
|---|---|---|
| Dispersogen® LFS | 2,1 | - |
| Propylenglykol | 4,3 | 4,3 |
| Defoamer® SE 57 | 0,6 | 0,6 |
| Kelzan® S (2 Gew.-% in Wasser) | 7,3 | 7,3 |
| Isosorbidcaprylat 1 | 0,3 | 0,2 |
| Benzyl Alcohol | 1,0 | 1,0 |
| Wasser | ad 100 | ad 100 |

Herstellung:

Der Wirkstoff wird mit den anderen Inhaltsstoffen (außer der Kelzan® S-Lösung) vordispergiert und anschließend einer Feinmahlung unterzogen, bis die mittlere Teilchengröße < 2 Mikrometer beträgt. Anschließend wird die Kelzan® S-Lösung eingerührt.

Formulierungsbeispiel 5: Handgeschirrspülmittel

| Inhaltsstoff | Gew. % |
|---|---|
| Hostapur® SAS 60 (Alkansulfonat, 60 Gew.-% in Wasser) | 40,0 |
| Hostapur® OS liquid (Sodium C14-16 Alkyl Sulfonate, 40 Gew.-% in Wasser) | 11,0 |
| Genaminox® LA (Dimethyllauraminoxid, 30 Gew.-% in Wasser) | 3,0 |
| Genagen® CAB (Cocoamidopropylbetain, 30 Gew.-% in Wasser) | 3,0 |
| Isosorbidcaprylat 1 | 0,8 |
| Benzyl Alcohol | 0,8 |
| Wasser | ad 100 |

Formulierungsbeispiel 6: Oberflächenreiniger (Allzweckreiniger)

| Inhaltsstoff | Gew.-% |
|---|---|
| Hostapur® SAS 60 (Alkansulfonat, 60 Gew.-% in Wasser) | 5,0 |
| Genapol® UD 080 (Undecanol + 8 EO) | 2,0 |
| Genaminox® LA (Dimethyllauraminoxid, 30 Gew.-% in Wasser) | 2,0 |
| Methylisothiazolinone | 0,01 |
| Isosorbidcaprylat 1 | 1,0 |
| Wasser | ad 100 |

Herstellung der Formulierungsbeispiele 5 und 6:

Die Hälfte der Wassermenge wird vorgelegt und die Komponenten werden in der Reihenfolge wie in den bei Formulierungsbeispielen 5 und 6 angegebenen Tabellen aufgeführt eingerührt. Die restliche Menge Wasser wird dann nachgegeben. Es resultieren klare, wässrige Zusammensetzungen.

## Patentansprüche

1. Verwendung einer oder mehrerer der Verbindungen der Formel (I)

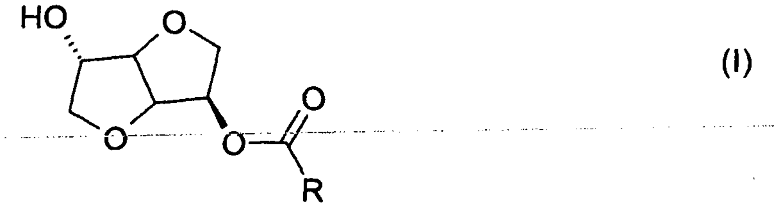

(I)

worin

R eine lineare oder verzweigte, gesättigte Alkylgruppe mit 5 bis 11 Kohlenstoffatomen oder eine lineare oder verzweigte, ein- oder mehrfach ungesättigte Alkenylgruppe mit 5 bis 11 Kohlenstoffatomen ist,

als Fungizid.

2. Verwendung nach Anspruch 1, **dadurch gekennzeichnet, dass** der Rest R in Formel (I) ein linearer gesättigter Alkylrest mit 7 bis 9 Kohlenstoffatomen ist.

3. Verwendung nach Anspruch 2, **dadurch gekennzeichnet, dass** der Rest R in Formel (I) ein linearer gesättigter Alkylrest mit 7 Kohlenstoffatomen ist.

4. Verwendung nach einem oder mehreren der Ansprüche 1 bis 3, **dadurch gekennzeichnet, dass** die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbiddiestern und Carbonsäuren verwendet werden.

5. Verwendung nach Anspruch 4, **dadurch gekennzeichnet, dass** die Zusammensetzung eine oder mehrere Verbindungen der Formel (I) und zusätzlich

I) Isosorbid und
II) einen oder mehrere Isosorbiddiester der Formel (II)

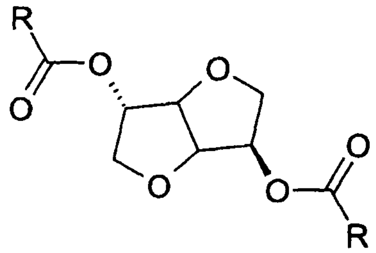

(II)

worin R die bei Formel (I) angegebene Bedeutung besitzt, enthält.

6. Verwendung nach Anspruch 4 oder 5, **dadurch gekennzeichnet, dass** die Zusammensetzung eine oder mehrere Verbindungen der Formel (I) und zusätzlich

I) 0,05 bis 0,7 Gewichtsteile Isosorbid und
II) 0,1 bis 1,0 Gewichtsteile an dem einem oder den mehreren Isosorbiddiestern der Formel (II)

enthält, jeweils bezogen auf 1,0 Gewichtsteile an der einen oder den mehreren Verbindungen der Formel (I).

7. Verwendung nach einem oder mehreren der Ansprüche 4 bis 6, **dadurch gekennzeichnet, dass** die Zusammensetzung eine oder mehrere Verbindungen der Formel (I) und einen oder mehrere Sorbitanester aus Sorbitan und

Carbonsäuren $R^aCOOH$, wobei $R^a$ eine lineare oder verzweigte, gesättigte Alkylgruppe mit 5 bis 11 Kohlenstoffatomen oder eine lineare oder verzweigte, ein- oder mehrfach ungesättigte Alkenylgruppe mit 5 bis 11 Kohlenstoffatomen ist, enthält, und das Gewichtsverhältnis der einen oder der mehreren Verbindungen der Formel (I) zu dem einen oder den mehreren soeben genannten Sorbitanestern von 70 : 30 bis 100 : 0 ist.

**8.** Verwendung nach Anspruch 7, **dadurch gekennzeichnet, dass** der eine oder die mehreren Sorbitanester aus Sorbitan und Carbonsäuren $R^aCOOH$ ausgewählt sind aus Sorbitanestern aus Sorbitan und Caprylsäure.

**9.** Verwendung nach einem oder mehreren der Ansprüche 4 bis 8, **dadurch gekennzeichnet, dass** die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbiddiestern und Carbonsäuren in der Zusammensetzung kleiner oder gleich 320 ist.

**10.** Verwendung nach einem oder mehreren der Ansprüche 4 bis 9, **dadurch gekennzeichnet, dass** die Zusammensetzung die eine oder die mehreren Verbindungen der Formel (I) in Mengen von mindestens 30 Gew.-% enthält, jeweils bezogen auf das Gesamtgewicht der fertigen Zusammensetzung.

**11.** Verwendung nach einem oder mehreren der Ansprüche 1 bis 10 in kosmetischen, dermatologischen oder pharmazeutischen Zusammensetzungen, in Pflanzenschutzformulierungen, in Wasch- oder Reinigungsmitteln oder in Farb- oder Anstrichmitteln.

**12.** Verwendung nach Anspruch 11, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel die eine oder die mehreren Verbindungen der Formel (I) in Mengen von 0,01 bis 10,0 Gew.-% enthalten, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

**13.** Verwendung nach Anspruch 11 oder 12, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel auf wässriger oder wässrig-alkoholischer Basis aufgebaut sind oder als Emulsion oder Dispersion vorliegen und vorzugsweise als Emulsion vorliegen.

**14.** Verwendung nach einem oder mehreren der Ansprüche 11 bis 13, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel einen pH-Wert von 2 bis 11 besitzen.

## Claims

**1.** Use of one or more compounds of the formula (I)

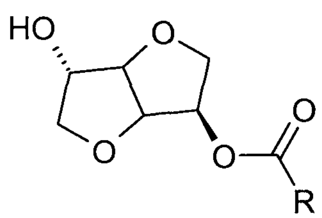

(I)

in which

R is a straight-chain or branched saturated alkyl group having 5 to 11 carbon atoms or a straight-chain or branched mono- or polyunsaturated alkenyl group having 5 to 11 carbon atoms

as fungicide.

**2.** The use as claimed in claim 1, wherein the radical R in formula (I) is a straight-chain saturated alkyl radical having

7 to 9 carbon atoms.

**3.** The use as claimed in claim 2, wherein the radical R in formula (I) is a straight-chain saturated alkyl radical having 7 carbon atoms.

**4.** The use as claimed in one or more of claims 1 to 3, wherein the one or more compounds of the formula (I) are used in a composition comprising one or more other substances selected from the group consisting of sorbitol, sorbitol esters, sorbitan, sorbitan esters, isosorbide, isosorbide diesters and carboxylic acids.

**5.** The use as claimed in claim 4, wherein the composition comprises one or more compounds of the formula (I) and additionally

I) isosorbide and
II) one or more isosorbide diesters of the formula (II)

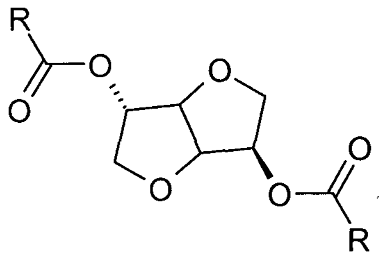

(II)

where R has the meaning given for formula (I).

**6.** The use as claimed in claim 4 or. 5, wherein the composition comprises one or more compounds of the formula (I) and additionally

I) from 0.05 to 0.7 part by weight of isosorbide and
II) from 0.1 to 1.0 part by weight of the one or more isosorbide diesters of the formula (II),

in each case based on 1.0 part by weight of the one or more compounds of the formula (I).

**7.** The use as claimed in one or more of claims 4 to 6, wherein the composition comprises one or more compounds of the formula (I) and one or more sorbitan esters of sorbitan and carboxylic acids RaCOOH, where Ra is a straight-chain or branched saturated alkyl group having 5 to 11 carbon atoms or a straight-chain or branched mono- or polyunsaturated alkenyl group having 5 to 11 carbon atoms, and where the weight ratio of the one or more compounds of the formula (I) to the one or more sorbitan esters just mentioned is from 70 : 30 to 100 : 0.

**8.** The use as claimed in claim 7, wherein the one or more sorbitan esters of sorbitan and carboxylic acids RaCOOH are selected from sorbitan esters of sorbitan and caprylic acid.

**9.** The use as claimed in one or more of claims 4 to 8, wherein the hydroxyl value of the mixture of the one or more compounds of the formula (I) and the one or more compounds selected from the group consisting of sorbitol, sorbitol esters, sorbitan, sorbitan esters, isosorbide, isosorbide diesters and carboxylic acids in the composition is smaller than or equal to 320.

**10.** The use as claimed in one or more of claims 4 to 9, wherein the composition comprises the one or more compounds of the formula (I) in amounts of at least 30% by weight, in each case based on the total weight of the finished composition.

**11.** The use as claimed in one or more of claims 1 to 10 in cosmetic, dermatological or pharmaceutical compositions, in crop protection formulations, in washing or cleaning compositions or in paints or coatings.

**12.** The use as claimed in claim 11, wherein the cosmetic, dermatological or pharmaceutical compositions, the crop protection formulations, the washing or cleaning compositions or the paints or coatings comprise the one or more

compounds of the formula (I) in amounts of from 0.01 to 10.0% by weight, in each case based on the total weight of the finished cosmetic, dermatological or pharmaceutical compositions, crop protection formulations, washing or cleaning compositions or paints or coatings.

**13.** The use as claimed in claim 11 or 12, wherein the cosmetic, dermatological or pharmaceutical compositions, the crop protection formulations, the washing or cleaning compositions or the paints or coatings are formulated on an aqueous or aqueous-alcoholic basis or are present as emulsion or dispersion, preferably as emulsion.

**14.** The use as claimed in one or more of claims 11 to 13, wherein the cosmetic, dermatological or pharmaceutical compositions, the crop protection formulations, the washing or cleaning compositions or the paints or coatings have a pH of from 2 to 11.

**Revendications**

**1.** Utilisation d'un ou de plusieurs des composés de formule (I)

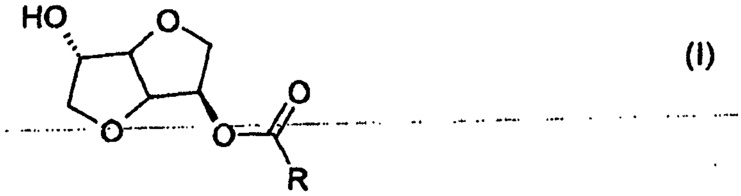

(I)

dans laquelle

R représente un groupe alkyle linéaire ou ramifié, saturé, de 5 à 11 atomes de carbone, ou un groupe alcényle linéaire ou ramifié, mono- ou polyinsaturé, de 5 à 11 atomes de carbone,

en tant que fongicide.

**2.** Utilisation selon la revendication 1, **caractérisée en ce que** le radical R dans la formule (I) est un radical alkyle linéaire saturé de 7 à 9 atomes de carbone.

**3.** Utilisation selon la revendication 2, **caractérisée en ce que** le radical R dans la formule (I) est un radical alkyle linéaire saturé de 7 atomes de carbone.

**4.** Utilisation selon une ou plusieurs des revendications 1 à 3, **caractérisée en ce que** le ou les composés de formule (I) sont utilisés dans une composition contenant une ou plusieurs autres substances choisies dans le groupe constitué par le sorbitol, les esters de sorbitol, le sorbitane, les esters de sorbitane, l'isosorbide, les diesters d'isosorbide et les acides carboxyliques.

**5.** Utilisation selon la revendication 4, **caractérisée en ce que** la composition contient un ou plusieurs composés de formule (I) et également

I) de l'isosorbide et
II) un ou plusieurs diesters d'isosorbide de formule (II)

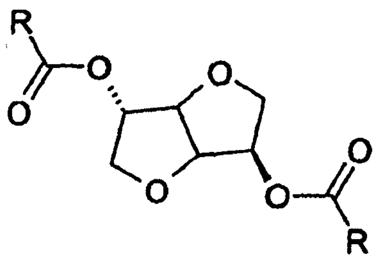

(II)

dans laquelle R a la signification indiquée pour la formule (I).

6. Utilisation selon la revendication 4 ou 5, **caractérisée en ce que** la composition contient un ou plusieurs composés de formule (I) et également

   I) 0,05 à 0,7 partie en poids d'isosorbide et
   II) 0,1 à 1,0 partie en poids du ou des diesters d'isosorbide de formule (II),

   à chaque fois par rapport à 1,0 partie en poids du ou des composés de formule (I).

7. Utilisation selon une ou plusieurs des revendications 4 à 6, **caractérisée en ce que** la composition contient un ou plusieurs composés de formule (I) et un ou plusieurs esters de sorbitane constitués de sorbitane et d'acides carboxyliques $R^aCOOH$, $R^a$ étant un groupe alkyle linéaire ou ramifié, saturé, de 5 à 11 atomes de carbone, ou un groupe alcényle linéaire ou ramifié, mono- ou polyinsaturé, de 5 à 11 atomes de carbone, et le rapport en poids entre le ou les composés de formule (I) et ledit ou lesdits esters de sorbitane étant de 70:30 à 100:0.

8. Utilisation selon la revendication 7, **caractérisée en ce que** le ou les esters de sorbitane constitués de sorbitane et d'acides carboxyliques $R^aCOOH$ sont choisis parmi les esters de sorbitane constitués de sorbitane et d'acide caprylique.

9. Utilisation selon une ou plusieurs des revendications 4 à 8, **caractérisée en ce que** l'indice OH du mélange du ou des composés de formule (I) et du ou des composés choisis dans le groupe constitué par le sorbitol, les esters de sorbitol, le sorbitane, les esters de sorbitane, l'isosorbide, les diesters d'isosorbide et les acides carboxyliques dans la composition est inférieur ou égal à 320.

10. Utilisation selon une ou plusieurs des revendications 4 à 9, **caractérisée en ce que** la composition contient le ou les composés de formule (I) en quantités d'au moins 30 % en poids, à chaque fois par rapport au poids total de la composition finie.

11. Utilisation selon une ou plusieurs des revendications 1 à 10 dans des compositions cosmétiques, dermatologiques ou pharmaceutiques, dans des formulations phytosanitaires, dans des détergents ou dans des colorants ou des peintures.

12. Utilisation selon la revendication 11, **caractérisée en ce que** les compositions cosmétiques, dermatologiques ou pharmaceutiques, les formulations phytosanitaires, les détergents ou les colorants ou les peintures contiennent le ou les composés de formule (I) en quantités de 0,01 à 10,0 % en poids, à chaque fois par rapport au poids total des compositions cosmétiques, dermatologiques ou pharmaceutiques, des formulations phytosanitaires, des détergents ou des colorants ou des peintures finis.

13. Utilisation selon la revendication 11 ou 12, **caractérisée en ce que** les compositions cosmétiques, dermatologiques ou pharmaceutiques, les formulations phytosanitaires, les détergents ou les colorants ou les peintures sont à base d'eau ou d'eau-alcool, ou se présentent sous la forme d'une émulsion ou d'une dispersion, et de préférence sous la forme d'une émulsion.

14. Utilisation selon une ou plusieurs des revendications 11 à 13, **caractérisée en ce que** les compositions cosmétiques, dermatologiques ou pharmaceutiques, les formulations phytosanitaires, les détergents ou les colorants ou les peintures présentent un pH de 2 à 11.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102009022444
- DE 102009022445, Clariant
- JP 8187070 A, Lion